# EUROPEAN PATENT SPECIFICATION

(11) **EP 2 604 592 B1**
(45) Date of publication and mention of the grant of the patent: **08.10.2014**
(21) Application number: 11460063.8
(22) Date of filing: 12.12.2011
(51) Int. Cl.: C07D 207/27

(54) **A process for the purification of aniracetam by crystallisation from aqueous solutions**
Verfahren zur Reinigung von Aniracetam durch Kristallisation aus wässrigen Lösungen
Procédé de purification d'aniracetam par cristallisation de solutions aqueuses

(43) Date of publication of application: 19.06.2013
(73) Proprietor: Zaklady Farmaceutyczne Polpharma SA, 83-200 Starogard Gdanski (PL)
(72) Inventor: Szramka, Roman, 83-140 Gniew (PL); Sagol, Karol, 83-110 Tczew (PL); Poojari, Krishna, 83-200 Starogard Gdanski (PL)

(56) References cited:
- EP-A1- 0 005 143
- RO-B- 113 039
- SMILOWSKI B.: "Trwalosc pochodnych 2-pirolidynonu II. Rozklad aniracetamu i jego produktu hydrolizy 2-pirolidonu w roztworach wodnych", ACTA POLONIAE PHARMACEUTICS, vol. 48, no. 5-6, 1 January 1991 (1991-01-01), pages 11-14, XP009157929,

## Description

### Field of the Invention

The invention concerns a new process for the purification of aniracetam which may be used on an industrial scale.

### Background of the Invention

Aniracetam is a racemic compound and it is used for the treatment of elderly patients with attention and memory disorders with vascular or degenerative aetiology.

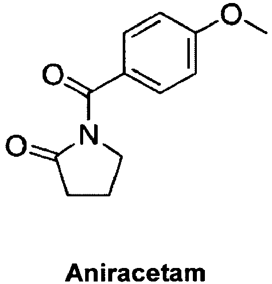

Aniracetam and the processes for the preparation thereof are disclosed in patent EP 005 143. The patent claims five different processes for the preparation of aniracetam:
a) acylation of 2-pyrrolidone in position 1,
b) methylation of 1-(p-hydroxybenzoyl)-2-pyrrolidone,
c) reduction of pyrrolin-2-one derivatives,
d) cyclisation of 4-(p-methoxybenzoylamino)butyric acid,
e) hydrolysis of 1-(p-methoxybenzoyl)-2-alkyloxypyrroline.

In examples whereby water or its aqueous solutions are used for product isolation, aniracetam is contacted with the solutions at temperatures close to 0 °C. The resulting crude product is generally re-crystallised from alcohol.

As B. 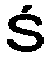 milowski discloses reports (Acta Poloniae Pharmaceutica (1991), 48(5-6), 11-14) aniracetam when contacted with water is hydrolysed throughout the pH range. The process of hydrolytic degradation of aniracetam in the acidic, neutral and alkaline environment is a first order consecutive reaction in which the final degradation products are: p-methoxybenzoic acid and 4-aminobutyric acid, with 2-pyrrolidone being the intermediate.

The requirements set forth by the International Conference on Harmonisation (ICH) with respect to active pharmaceutical ingredients allowed to be administered to humans require that new and more efficient methods for the reduction of impurities in active substances should be sought.

The required level of aniracetam purity is considered as preparation of a white product with a content of any single impurity below 0.10%.

The methods for the purification of aniracetam by re-crystallisation from alcohols known in the art do not enable the achievement of the required purity level (Table 1, Examples 2-4).

### Brief description of the Invention

It has unexpectedly been found that owing to the crystallisation of aniracetam from boiling aqueous solutions a white product with a content of any single impurity <_ 0.05% can be obtained with a high yield. The process conditions of invention enable the reduction of the unfavourable hydrolytic degradation of aniracetam which can lead to unwanted consequences like reduced yield and increased amount of impurities.

### Detailed description of the Invention

The present invention includes an efficient process for the purification of crude aniracetam as a result of which an active substance with the required purity level is obtained. Crude aniracetam can be obtained using methods known in the art or using the process disclosed in Example 1.

Using the process of the invention, crude aniracetam is purified by crystallisation at high temperatures from water or a mixture of water and alcohol, whereby the content of the alcohol in water does not exceed 50% by volume.

The unwanted hydrolytic degradation of aniracetam can be reduced by decreasing the time of residence in the aqueous solution at high temperature. In order to achieve this, crude aniracetam is introduced into the process when the water was heated to at least 50°C. Furthermore, the process is conducted in a way, such that the total time of 1) heating the aniracetam solution to a high temperature, 2) decolourisation with activated carbon and 3) filtration of the heated solution is shorter than 2 hours. The yield of aniracetam purification using the process of the invention is comparable and higher than that of re-crystallisation from alcohols (Table 1).

The term "high temperature" means a temperature of aniracetam solution at 70-80 °C or boiling temperature.

The term "hot water" means temperature of water at 50-60 °C.

The alcohols used in the process are aliphatic C₁₋₄ alcohols or mixture thereof.

The invention is illustrated by the following embodiments which in no way restrict its scope.

### Analytical methods:

In the embodiments of the invention below, the purity of the resulting product was determined using UPLC, performed With a liquid chromatograph adapted for Ultra-Performance Liquid Chromatography. A C18 column with packing with a grain size of 1.7 µm and gradient elution was used.

### Example 1. A process for the preparation of crude aniracetam.

94 g (0.79 mol) of thionyl chloride and 30 g (0.197) of 4-methoxybenzoic acid was measured into a 250 mL round-bottom flask fitted with a stirrer, thermometer and reflux condenser. Subsequently, the mixture was heated to a boil (76°C) and maintained at that temperature for three hours upon stirring; subsequently, the unreacted thionyl chloride was distilled off in vacuum. The distillation residue was cooled to 50°C and dissolved in 120 mL of toluene. 26.0 g (0.256 mol) of triethylamine and 17.6 g (0.207 mol) of 2-pyrrolidone was added to the toluene solution. The reaction mixture was heated to a boil (110°C) and maintained under reflux for 2.5 hours; subsequently, it was cooled to 20°C and 60 mL of water was added. The precipitate of crude aniracetam was filtered off, washed three times with 50 mL of water and dried. 34.9 g of a crude product was obtained with a yield of 80%.

### Reference examples 2-4. Purification of aniracetam by crystallisation from alcohol.

120 mL of alcohol and 1 g of activated carbon was measured into a 250 mL round-bottom flask fitted with a stirrer, thermometer and reflux condenser. The content of the flask was heated to 50°C upon stirring and 20 g (0.91 mol) of crude aniracetam prepared according to the process disclosed in Example 1 was added to the suspension at that temperature. The content was heated to a boil and maintained under reflux for 10 min. The hot suspension was filtered through a diatomaceous earth filter cake (such as HyfloSuper Cel); subsequently, the clear alcohol aniracetam solution was cooled to 20°C. The precipitate of aniracetam was filtered off and washed with 20 mL of alcohol. The product was dried in a vacuum dryer at a temperature of 50°C for 16 hours; beige-pink crystals were obtained.

The content of impurities was determined using UPLC (Table 1).

**Table 1**

| **Aniracetam** | **Solvent** | **Colour of the precipitate** | **Purity UPLC [%]** | **Assay UPLC [%]** | **Product mass** | **Yield [%]** |
|---|---|---|---|---|---|---|
| **Ex 1** *(crude)* | Toluene/ water | Beige-grey | Single impurity = **0.26** | 99.31 | - | 75-85 |
| ***REx 2** | MeOH | Beige-pink | Single impurity = 0.21 | 99.68 | 15.23 | 76.2 |
| ***REx 3** | EtOH | Beige-pink | Single impurity = 0.20 | 99.67 | 15.34 | 76.7 |
| ***REx 4** | iPrOH | Beige-pink | Single impurity = 0.23 | 99.61 | 15.24 | 76.2 |
| **Ex 5** | Water | White | Single impurity ≤ **0.05** | **99.92** | 22.5 | 74.9 |
| **Ex 6** | Water/Me OH | White | Single impurity < **0.10** | **99.90** | 23.1 | 82.0 |

| | | | | | | |
|---|---|---|---|---|---|---|
| * Reference example | | | | | | |

### Example 5. Purification of aniracetam by crystallisation from water.

1200 mL of water and 1.5 g of activated carbon was measured into a 1500 mL round-bottom flask fitted with a stirrer, thermometer and reflux condenser. The content of the flask was heated to 50°C upon stirring and pH was adjusted using NaHCO₃ solution between 6.8 and 7.2. Subsequently, 30 g (1.37 mol) of crude aniracetam prepared according to the process disclosed in Example 1 was added to the suspension. pH was adjusted with NaHCO₃ solution between 6.8 and 7.2 while the suspension was heated to a boil. The content of the flask was maintained under reflux for 5 min; subsequently, the hot suspension was filtered through a diatomaceous earth filter cake (such as HyfloSuper Cel), and the clear filtrate was cooled to 20°C, with pH being constantly adjusted between 6.8 and 7.2. The precipitate of aniracetam was filtered off and washed twice with 100 and 200 mL of water. The product was dried in a vacuum dryer at a temperature of 50°C for 16 hours; 22.5 g of white precipitate was obtained with a yield of 75%.

The content of impurities was determined using UPLC. No impurity exceeded 0.05% (Table 1 ).

### Example 6. Purification of aniracetam by crystallisation from water/methanol solution

900 mL of water and 1.5 g of activated carbon was measured into a 1500 mL round-bottom flask fitted with a stirrer, thermometer and reflux condenser. The content of the flask was heated to 50°C upon stirring and 30 g (1.37 mol) of crude aniracetam prepared according to the process disclosed in Example 1 was added to the suspension at that temperature. Subsequently, 300 mL of hot (60°C) methanol was added to the flask and the content was heated to a temperature of 75°C. The content was stirred for 30 min at a temperature of 75°C; subsequently, the hot suspension was filtered through a diatomaceous earth filter cake (such as HyfloSuper Cel) and the clear filtrate was cooled to 20°C. The precipitate of aniracetam was filtered off and washed twice with 150 mL of a water/methanol (3:1) mixture and, subsequently, twice with 200 mL of water. The product was dried in a vacuum dryer at a temperature of 50°C for 16 hours; 23.1 g of white precipitate was obtained with a yield of 82%.

The content of impurities was determined using UPLC. No impurity exceeded 0.10% (Table 1).

## Claims

1. A process for the purification of aniracetam by crystallisation from aqueous solutions which comprises at least the following steps:
b) crude aniracetam is added to water at temperature of 50-60°C and pH is optionally adjusted to neutral, or
crude aniracetam is added to water at temperature of 50-60°C and followed by adding of alcohol,
b) the aqueous aniracetam solution is heated to temperature at least 70 °C and decolourised with activated carbon,
c) aniracetam is precipitated by cooling the solution to room temperature,

2. A process according to Claim 1, **characterised in that** the alcohol content in the aqueous solution is equal to or less than 50% by volume.

3. A process according to Claim 1, **characterised in that** the total time of retaining the aniracetam solution at a temperature above 50°C is shorter than 2 hours.

4. A process according to Claim 1, **characterised in that** an alcohol used in the process belong to group of aliphatic alcohols C₁₋₄ or mixture thereof.

## Patentansprüche

1. Verfahren zur Aufreinigung von Aniracetam durch Kristallisieren aus wässrigen Lösungen, welches mindestens die folgenden Schritte umfasst:
a) rohes Aniracetam wird zu Wasser mit einer Temperatur von 50-60°C gegeben, und der pH-Wert wird gegebenenfalls auf neutral eingestellt, oder rohes Aniracetam wird zu Wasser mit einer Temperatur von 50-60°C gegeben, gefolgt von der Zugabe von Alkohol,
b) die wässrige Aniracetamlösung wird auf eine Temperatur von mindestens 70°C erhitzt und mit Aktivkohle entfärbt,
c) Aniracetam wird durch Abkühlen der Lösung auf Raumtemperatur ausgefällt.

2. Verfahren nach Anspruch 1, **dadurch gekennzeichnet, dass** der Alkoholgehalt in der wässrigen Lösung gleich 50 Vol.-% ist oder darunter liegt.

3. Verfahren nach Anspruch 1, **dadurch gekennzeichnet, dass** die Gesamtverweilzeit der Aniracetanlösung bei einer Temperatur über 50°C unter 2 Stunden liegt.

4. Verfahren nach Anspruch 1, **dadurch gekennzeichnet, dass** der in dem Verfahren eingesetzte Alkohol zur aus aliphatischen C₁₋₄-Alkoholen und Mischungen davon bestehenden Gruppe gehört.

## Revendications

1. Procédé de purification d'aniracétam par crystallisation à partir de solutions aqueuses, comprenant au moins les étapes suivantes :
a) de l'aniracétam brut est ajouté à de l'eau à une température de 50-60°C et le pH est éventuellement ajusté à un pH neutre, ou
de l'aniracétam brut est ajouté à de l'eau à une température de 50-60 °C, suivi de l'addition d'un alcool,
b) la solution d'aniracétam aqueuse est chauffée à une température d'au moins 70°C, et décolorée par du charbon actif,
c) l'aniracétam est précipité en refroidissant la solution jusqu'à température ambiante.

2. Procédé selon la revendication 1, **caractérisé en ce que** la teneur en alcool dans la solution aqueuse est égale ou inférieure à 50% en volume.

3. Procédé selon la revendication 1, **caractérisé en ce que** le temps total de rétention de la solution d'aniracétam à une température supérieure à 50°C est inférieure à 2 heures.

4. Procédé selon la revendication 1, **caractérisé en ce qu'**un alcool utilisé dans le procédé appartient au groupe des alcools aliphatiques en C₁₋₄ ou un mélange de ceux-ci.
